# FASCICULE DE BREVET EUROPEEN

(11) **EP 1 016 669 B1**
(45) Date de publication et mention de la délivrance du brevet: **19.02.2003**
(21) Numéro de dépôt: 99402783.7
(22) Date de dépôt: 09.11.1999
(51) Int. Cl.: C07H 17/08, C07D 233/64, A61K 31/70, A61P 31/04

(54) **Nouveaux dérivés de l'érythromycine, leur procédé de préparation et leur application comme médicaments**
Erythromycin-Derivate, Verfahren zu deren Herstellung und deren Verwendung als Arzneimittel
New derivatives of erythromycin, process for making them as well as their use as medicaments

(30) Priorité: 10.11.1998 FR 9814145
(43) Date de publication de la demande: 05.07.2000
(73) Titulaire: Aventis Pharma S.A., 92160 Antony (FR)
(72) Inventeur: Agouridas, Constantin, 94130 Nogent sur Marne (FR); Denis, Alexis, 75011 Paris (FR); Fromentin, Claude, 75019 Paris (FR)

(56) Documents cités:
- EP-A- 0 596 802
- WO-A-98/09978
- WO-A-98/40392
- FR-A- 2 669 337
- FR-A- 2 742 757
- DENIS A ET AL: "Synthesis of 6-O-methyl-azithromycin and its ketolide analogue via Beckmann rearrangement of 9(E)-6-O-methyl-erythromycin oxime" BIOORGANIC & MEDICINAL CHEMISTRY LETTERS, vol. 8, no. 18, 22 septembre 1998 (1998-09-22), page 2427-2432 XP004138245 ISSN: 0960-894X

## Description

La présente invention concerne de nouveaux dérivés de l'érythromycine, leur procédé de préparation et leur application comme médicaments.

L'état de la technique est essentiellement constitué par les documents suivants :

La demande internationale WO 98 09978 décrit notamment des dérivés 3-oxo de l'érythromycine comportant en 2 le seul radical méthyl, en 6 un méthyl substitué et en 12,13 un groupement -O-L-T- dans lequel L est notamment un carbonyl et T un groupement -N(W-Rd)- dans lequel W peut être absent et Rd peut représenter un hétéroaryl.

La demande française FR-A-2 742 757 décrit notamment des dérivés 3-oxo de l'érythromycine, comportant en 2 un méthyl et un halogène et en 12,13 un groupement carbamate substitué sur l'azote par un alkyl substitué par un aryl pouvant être hétérocyclique.

La demande française FR-A-2 669 337 décrit notamment des dérivés 3-oxo de l'érythromycine, comportant en 2 un méthyl et, le cas échéant, un groupe amino ou alkyl éventuellement substitué par un amino, et en 12,13 un groupement carbamate substitué sur l'azote par un alkyl substitué par un groupe amino ou mono ou dialkylamino.

La référence Biorganic and Medicinal Chemistry Letters 8(1998) 2427-2432 décrit la synthèse de kétolides connus par réarrangement de Beckmann à partir de l'oxime de 9(E)6-O-méthyl-érythromycine et notamment celle d'un dérivé comportant en 12,13 un groupement carbamate substitué sur l'azote par un radical 2-pyridyl imodazolyl butyl.

Enfin, la demande européenne EP 0 596 802 décrit notamment des dérivés 3-oxo de l'érythromycine, comportant en 12,13 un groupement carbamate substitué sur l'azote par un radical butyl substitué par un radical aryl hétérocyclique.

L'invention a pour objet à titre de produits chimiques nouveaux, les composés de formule (I) ainsi que leurs sels d'addition avec les acides : 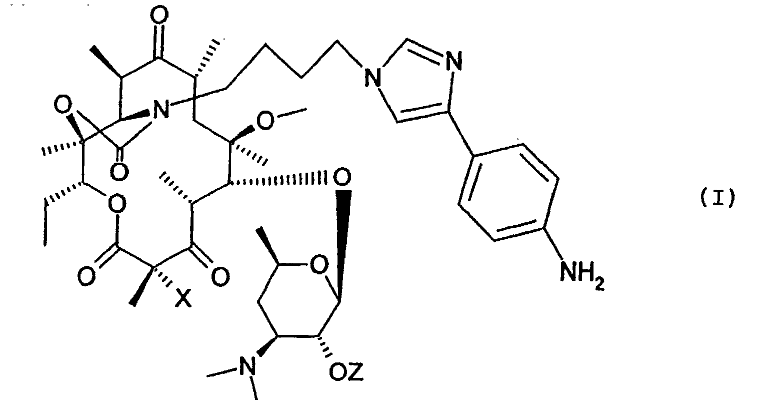 dans laquelle X représente un atome d'hydrogène ou un atome d'halogène et Z représente un atome d'hydrogène ou le reste d'un acide.

Parmi les sels d'addition avec les acides, on peut citer les sels formés avec les acides acétique, propionique, trifluoroacétique, maléique, tartrique, méthanesulfonique, benzènesulfonique, p-toluènesulfonique et spécialement les acides stéariques, éthylsuccinique ou laurylsulfonique.

L'atome d'halogène est par exemple un atome de chlore ou de fluor et de préférence un atome de fluor.

L'invention a plus particulièrement pour objet les composés de formule (I) dans lesquels Z représente un atome d'hydrogène.

L'invention a plus particulièrement pour objet les composés dont la préparation est donnée ci-après dans la partie expérimentale.

Les produits de formule (I) possèdent une bonne activité antibiotique sur les bactéries gram^{⊕} tels que les staphylocoques, les streptocoques, les pneumocoques.

Les produits sont particulièrement actifs sur les souches résistantes à l'érythromycine comme par exemple Streptococcus pyogenes et Streptococcus pneumoniae et S. aureus possédant une résistance inductible à l'érythromycine.

Les composés de l'invention peuvent donc être utilisés comme médicaments dans le traitement des infections à germes sensibles et, notamment, dans celui des staphylococcies, telles que les septicémies à staphylocoques, staphylococcies malignes de la face ou cutanées, pyodermites, plaies septiques ou suppurantes, furoncles, anthrax, phlegmons, érysipèles et acné, staphylococcies telles que les angines aiguës primitives ou post grippales, broncho-pneumonie, suppuration pulmonaires, les streptococcies telle que les angines aiguës, les otites, les sinusites, la scarlatine, les pneumococcies telles que les pneumonies, les bronchites et la diphtérie. Les produits de la présente invention sont également actifs contre les infections dues à des germes comme Haemophilus influenzae.

L'invention a donc pour objet les composés de formule (I) à titre de médicaments.

L'invention a plus spécialement pour objet à titre de médicaments les composés indiqués ci-dessus comme composés préférés.

L'invention a également pour objet les compositions pharmaceutiques renfermant comme principe actif au moins un des médicaments définis ci-dessus.

Ces compositions peuvent être administrées par voie buccale, rectale, parentérale ou par voie locale en application topique sur la peau et les muqueuses, mais la voie d'administration préférée est la voie buccale ou injectable. Elles peuvent être solides ou liquides et se présenter sous les formes pharmaceutiques couramment utilisées en médecine humaine, comme par exemple, les comprimés simples ou dragéifiés, les gélules, les granulés, les suppositoires, les préparations injectables, les pommades, les crèmes, les gels ; elles sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Ces compositions peuvent également se présenter sous forme d'une poudre destinée à être dissoute extemporanément dans un véhicule approprié, par exemple de l'eau stérile apyrogène.

La dose administrée est variable selon l'affection traitée, le sujet en cause, la voie d'administration et le produit considéré. Elle peut être, par exemple, comprise entre 50 mg et 3000 mg par jour par voie orale ou injectable, chez l'adulte pour les produits préférés.

L'invention a également pour objet un procédé de préparation caractérisé en ce que l'on soumet un composé de formule (II) 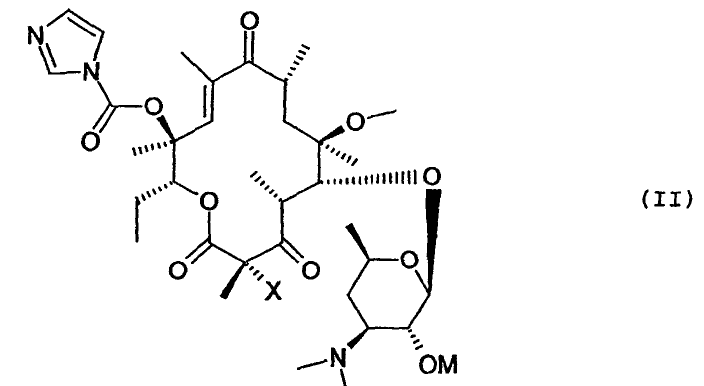 dans laquelle X conserve sa signification précédente et M représente le reste d'un radical acyl à l'action d'un composé de formule (III) 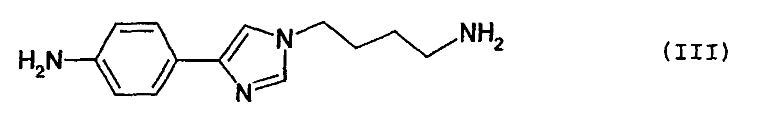 puis si désiré, on soumet le composé obtenu à l'action d'un agent de déprotection de la fonction hydroxyle en 2' et/ou le cas échéant, à l'action d'un acide pour en former le sel.
- la réaction du composé de formule (II) avec le composé de formule (III) a lieu au sein d'un solvant tel que par exemple l'acétonitrile, le diméthylformamide ou encore le tétrahydrofuranne, le diméthoxyéthane ou le diméthylsulfoxyde,
- l'hydrolyse de la fonction ester en 2' est réalisée à l'aide du méthanol ou de l'acide chlorhydrique aqueux,
- la salification est réalisée au moyen d'acides selon les procédés classiques.

Les composés de formule (II) dans lesquels X représente un atome d'hydrogène, utilisés comme produits de départ sont décrits et revendiqués dans la demande de brevet européen 0 596 802.

Les composés de formule (II) utilisés comme produits de départ dans lesquels X représente un atome de fluor peuvent être préparés comme indiqués ci-après dans la partie expérimentale.

Le composé III est un produit nouveau et est en lui-même un objet de la présente invention.

### EXEMPLE 1 : 11,12-didéoxy-3-de[(2,6-didéoxy-3-C-méthyl-3-0-méthyl-alpha-L-ribohexopyranosyl)oxy]-6-0-méthyl-3-oxo-12,11-[oxycarbonyl-[[4-[4-(4-aminophényl)-1H-imidazol-1-yl]butyl]imino]]-érythromycine

On agite à la température ambiante pendant 48 heures un mélange renfermant 0,690 g de produit de la préparation 1, 14 ml de THF, 14 ml d'isopropanol, 1,41 g de 2'-acétate et 12-[(1H-imidazol-1-yl)carboxylate]de 10,11-didéhydro-11-déoxy-3-de [(2,6-didéoxy-3-C-méthyl-3-0-méthyl-alpha-L-ribohexopyranosyl)oxy]-6-0-méthyl-3-oxo-érythromycine et 60 µl de DBU. On verse sur l'eau, extrait à l'acétate d'éthyle, lave à l'eau, sèche, filtre et concentre. On obtient 1,54 g de produit que l'on reprend dans 15 ml de méthanol additionné de 0,015 ml de DBU. On chasse le méthanol sous pression réduite et obtient 1,44 g de produit que l'on chromatographie sur silice en éluant avec le mélange chlorure de méthylène, méthanol ammoniaque 93-7-0,4. On obtient 0,84 g de produit que l'on reprend dans l'acétate d'éthyle, l'eau et l'ammoniaque. On extrait, sèche, filtre et concentre. On obtient 0,8 g de produit que l'on chromatographie sur silice en éluant avec le mélange acétate d'éthyle méthanol triéthylamine. On obtient 0,4 g de produit que l'on cristallise dans l'éther, essore et sèche. On obtient 0,270 g de produit recherché. F = 188 ^{~} 190°C.
Spectre de masse MH⁺ = 826⁺
**RMN CDCl**_{**3**} **ppm**

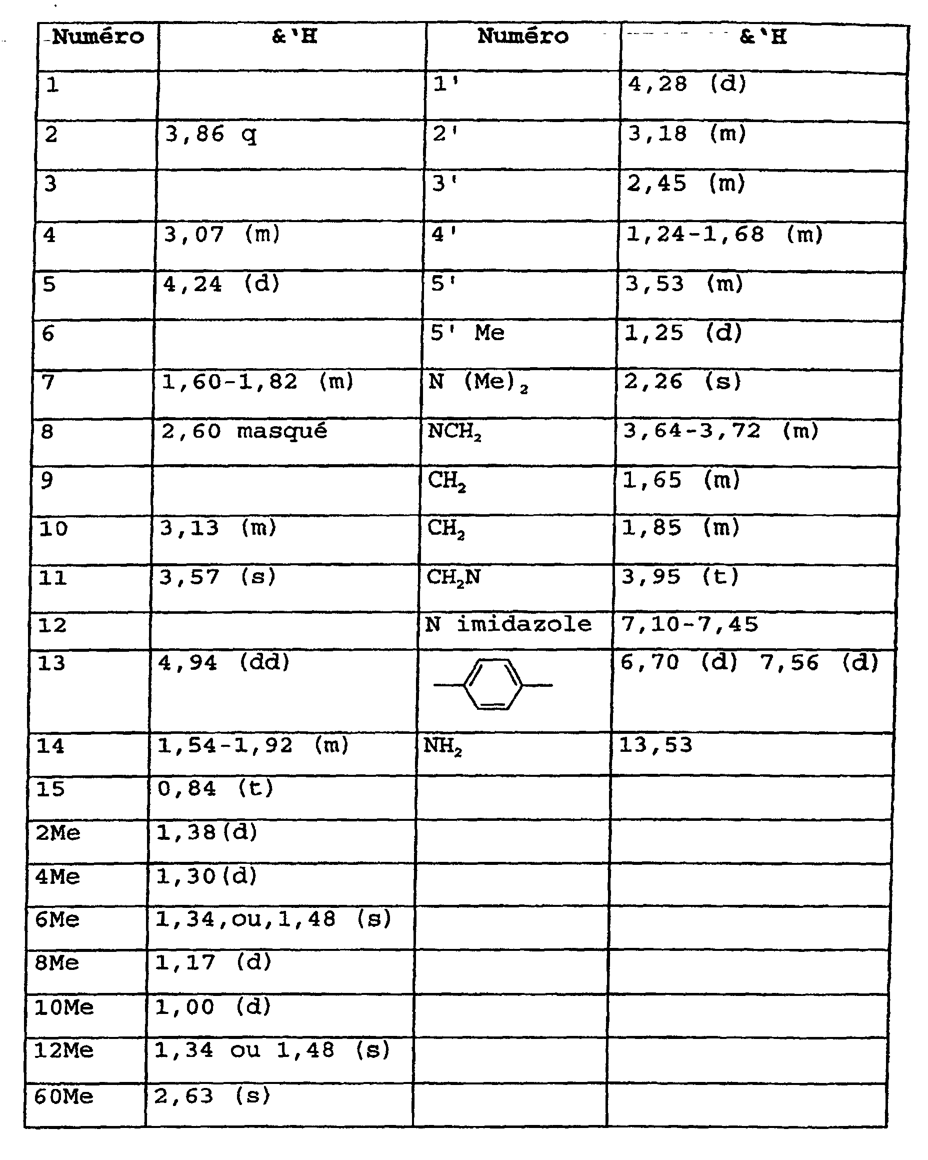

### Préparation 1

### 4-(4-aminophényl)-1H-imidazole-1-butanamine

### Stade A : 4-(4-nitrophényl)-1H-imidazole

On agite à 180°C pendant 1 heure 9,7 g de 2-bromo-1-(4-nitrophényl)-éthanone et 30 ml de formamide. On refroidit le milieu réactionnel et le verse dans l'eau. On amène à pH 1 à l'aide d'une solution d'acide chlorhydrique et extrait à l'acétate d'éthyle. La phase aqueuse est additionnée d'ammoniaque concentrée, saturée au chlorure de sodium et extraite à l'acétate d'éthyle. On sèche la phase organique, filtre et concentre sous pression réduite. On obtient 7,09 g de produit que l'on empâte dans l'éther diéthylique, essore et sèche. On obtient 4,74 g de produit fondant à 216 218°C.

### Stade B : 2-[4-[4-(4-nitrophényl)-1H-imidazole-1-yl]butyl]-1H-isoindole-1,3(2H)-dione.

On introduit une solution renfermant 4,7 g de produit du stade précédent et 15 ml de DMF dans un mélange renfermant 1,44 g d'hydrure de sodium et 12,5 ml de DMF. On ajoute une solution renfermant 7,05 g de N-(4-bromobutyl)phtalimide et 17,5 ml de DMF. On agite pendant 3 heures à la température ambiante. On verse le milieu réactionnel sur un mélange d'eau et de glace, extrait à l'acétate d'éthyle, lave à l'eau, sèche, filtre et concentre. On obtient 6,77 g de produit que l'on chromatographie sur silice en éluant avec le mélange acétate d'éthyle triéthylamine 95-5. On obtient 2,29 g de produit fondant à 170 ^{~} 172°C.

### Stade C : 2-[4-[4-(4-aminophényl]-1H-imidazole-1-yl]butyl]-1H-isoindole-1,3(2H)-dione.

On agite à la température ambiante sous pression d'hydrogène pendant 3 heures un mélange de 2 g de produit du stade précédent, 41 ml d'un mélange méthanol, chlorure de méthylène (20,5ml - 20,5 ml) et 200 mg de palladium à 10 % sur charbon. On filtre, lave avec un mélange chlorure de méthylène méthanol 50-50, et concentre sous pression réduite. On obtient 1,6 g de produit que l'on utilise tel quel dans le stade suivant.

### Stade D : 4-(4-aminophényl)-1H-imidazole-1-butanamine

On porte au reflux pendant 24 heures un mélange renfermant 1,6 g de produit du stade précédent, 1,1 ml d'hydrate d'hydrazine et 35,5 cm³ d'éthanol absolu. On refroidit le milieu réactionnel à la température ambiante, filtre, rince à l'éthanol, concentre sous pression réduite, reprend au chlorure de méthylène, filtre et concentre. On obtient 0,71 g de produit recherché.

### EXEMPLE 2

### 2-fluoro-11,12-didéoxy-3-de[(2,6-didéoxy-3-C-méthyl-3-0-méthyl-alpha-L-ribohexopyranosyl)oxy]-6-0-méthyl-3-oxo-12,11-[oxycarbonyl[[4-[4-(4-aminophényl)-1H-imidazol-1-yl]butyl]imino]]-érythromycine

En opérant comme précédemment à partir du produit de la préparation 2, on a obtenu le produit recherché.
CCM : acétate d'éthyle/triéthylamine 90-10. Rf 0,20

### Préparation 2

### 2'-acétoxy 2-α-fluoro de 12-(oxycarbonylimidazol)11-déoxy 10,11-didéhydro-3- de-[(2,6-didéoxy-3-C-méthyl-3-0-méthyl-α -L-ribohexopyranosyl)oxy]-6-0-méthyl-3-oxo-érythromycine.

### Stade A : 11-déoxy-10,11-didéhydro-3-de[(2,6-didéoxy-3-0-méthyl-α-L-ribohexopyranosyl)-oxy]-6-0-méthyl-3-oxo-erythromycine.

On agite pendant 44 heures un mélange de 8, 722 g de 2'-acétate de 11-déoxy 10,11-didéhydro 3-de[(2,6-didéoxy 3-0-méthyl α L-ribohexopyranosyl)oxy]6-0-méthyl 3-oxo érythromycine (EP 596802) et 350 ml de méthanol anhydre. On obtient 8,794 g du produit recherché.

### Stade B : 2'-triméthylsilyloxy de 11-déoxy-10,11-didéhydro-3-de [(2,6-didéoxy-3-0-méthyl-α-L-ribohexopyranosyl)oxy]-6-0-méthyl-3-oxo-érythromycine.

On agite à température ambiante pendant 4 jours un mélange renfermant 3,08 g du produit du stade précédent, 340 mg d'imidazole, 32 ml de THF anhydre et 1,06 ml d'hexaméthyldisilazane. On évapore à sec, reprend avec un mélange de 60 ml de chlorure de méthylène et de 60 ml de phosphate acide de sodium 0,5 M. On maintient le mélange réactionnel sous agitation pendant 15 minutes, décante, extrait au chlorure de méthylène, sèche et évapore à sec. On obtient 3,345 g du produit recherché.

### Stade C : 2'-triméthylsilyloxy 2α-fluoro de 11-déoxy-10,11-didéhydro-3- de-[(2,6-didéoxy-3-0-méthyl-α-L-ribohexopyranosyl)oxy]-6-0-méthyl-3-oxo-érythromycine.

On ajoute à - 12°C sous atmosphère d'argon 1,24 ml d'une solution de terbutylate de potassium dans le THF 0,97M dans une solution renfermant 668 mg de 2'-triméthylsilyloxy de 11-déoxy 10,11-didéhydro 3-de [(2,6-didéoxy 3-0-méthyl α-L-ribohexopyranosyl)oxy]6-0-méthyl 3-oxo érythromycine et 6,7 ml de THF anhydre. On agite 5 minutes et ajoute 378 mg de N-fluorodibenzènesulfonimide. On agite 10 minutes à -12°C et laisse revenir à la température ambiante pendant 1 heure 30 minutes. On effectue les opérations d'isolation et purification et obtient 695 mg du produit recherché.

### Stade D : 2α-fluoro de 11-déoxy-10,11-didéhydro-3- de [(2,6-didéoxy-3-0-méthyl-3-0-méthyl-α-L-ribohexopyranosyl)oxy]-6-0-méthyl-3-oxo-érythromycine

On agite pendant 3 heures 30 minutes un mélange de 5,476 g de produit du stade précédent, 50 ml de THF et 11,2 ml de fluorure de tétrabutylammonium 1M dans le THF. On évapore le solvant et ajoute 37 ml d'acétate d'éthyle, 37 ml d'eau et 7,5 ml d'ammoniaque à 20%. On agite 10 minutes, décante, extrait à l'acétate d'éthyle, sèche, filtre et concentre à sec le filtrat. On chromatographie le produit obtenu sur silice en éluant avec le mélange CH₂Cl₂-MeOH ammoniaqué 99-1, puis 98-2, 97-3, 96-4, 95-5. On obtient 2,452 g de produit recherché.

### Stade E : 2'-acétoxy-2α-fluoro de 11-déoxy-10,11-didéhydro 3- de [(2,6-didéoxy-3-0-méthyl-α-L-ribohexopyranosyl)oxy] 6-0-méthyl-3-oxo-érythromycine

On maintient sous agitation pendant 3 heures 1,02 g de produit du stade D, 10 ml de chlorure de méthylène et 241 µl d'anhydride acétique. On évapore et ajoute 10 ml d'eau et 10 ml d'acétate d'éthyle. On laisse 1 heure à la température ambiante sous agitation, décante, sèche et évapore. On obtient 1,01 g de produit recherché.

### Stade F : 2'-acékoxy-2α-fluoro de 12-(oxycarbonylimidazol) 11-déoxy-10,11-didéhydro-3- de-[(2,6-didéoxy-3-C-méthyl-3-0-méthyl-α-L-ribohexopyranosyl)oxy]-6-0-méthyl-3-oxo-érythromycine

On ajoute à 0°C 0,388 g de carbonyldiimidazole et 24 µl de DBU dans une solution renfermant 1,01 g du produit du stade précédent et 10 ml de THF anhydre. On évapore le THF et ajoute 10 ml d'eau et 10 ml d'acétate d'éthyle. On maintient le mélange réactionnel sous agitation pendant 10 minutes, extrait, sèche et évapore. On obtient 0,902 g de produit recherché brut que l'on chromatographie en éluant avec un mélange acétate d'éthyle-triéthylamine 96-4. On obtient 0,573 g de produit recherché.

### EXEMPLES DE COMPOSITIONS PHARMACEUTIQUES

On a préparé des comprimés renfermant :

| | |
|---|---|
| Produit de l'exemple 1 | 150 mg |
| Excipient q.s.p. | 1 g |
| Détail de l'excipient : amidon, talc, stéarate de magnésium Produit de l'exemple 2 | 150 mg |
| Excipient q.s.p. | 1 g |

Détail de l'excipient : amidon, talc, stéarate de magnésium On a également préparé des solutions injectables à partir des composés salifiés.

### ETUDE PHARMACOLOGIQUE DES PRODUITS DE L'INVENTION

### A - Méthode des dilutions en milieu liquide

On a préparé une série de tubes dans lesquels on répartit une même quantité de milieu nutritif stérile. On distribue dans chaque tube des quantités croissantes du produit à étudier, puis chaque tube est ensemencé avec une souche bactérienne. Après incubation de 24 heures à l'étuve à 37 °C, l'inhibition de la croissance est appréciée par transillumination de ce qui permet de déterminer les concentrations minimales inhibitrices exprimés en microgrammes/cm³. Les résultats suivants ont été obtenus.

| | **EX.1** | **EX.2** |
|---|---|---|
| Streptococcus pyogenes | 2,5 | 0,3 |
| Streptococcus pneumoniae | 1,2 | 0,150 |

## Revendications

1. A titre de produits chimiques les composés de formule (I) ainsi que leurs sels d'addition avec les acides : 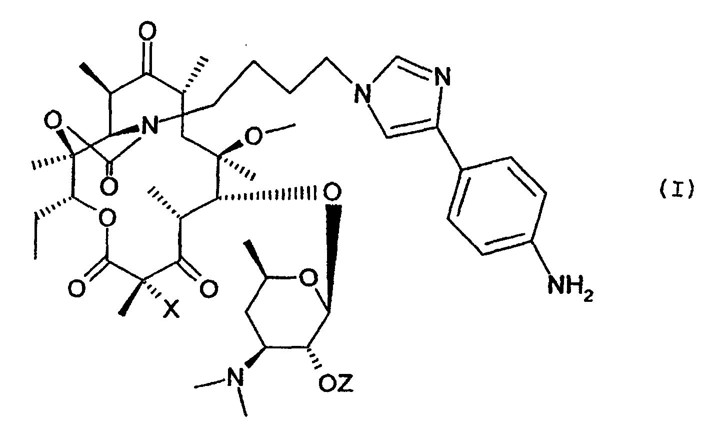 dans laquelle X représente un atome d'hydrogène ou un atome d'halogène et Z représente un atome d'hydrogène ou le reste d'un acide.

2. Les composés de formule (I) dans lesquels X représente un atome de fluor.

3. Les composés de formule (I) définis à la revendication 1 ou 2 dans lesquels Z représente un atome d'hydrogène.

4. A titre de produit chimique défini à la revendication 1, le composé suivant :
11,12-didéoxy-3-de [(2,6-didéoxy-3-C-méthyl-3-O-méthyl-alpha-L-ribohexopyranosyl)oxy] 6-0-méthyl 3-oxo 12,11-[oxycarbonyl-[[4-[4-(4-aminophényl)-1H-imidazol-1-yl]-butyl]-imino]]-érythromycine.

5. A titre de produit chimique défini à la revendication 1 le composé suivant :
2-fluoro-11,12-didéoxy-3-de [(2,6-didéoxy-3-C-méthyl-3-O-méthyl-alpha-L-ribohexopyranosyl)oxy] 6-0-méthyl 3-oxo 12,11-[oxycarbonyl [[4-[4-(4-aminophényl)-1H-imidazol-1-yl]-butyl]-imino]]-érythromycine.

6. Procédé de préparation des composés de formule (I) défini à l'une quelconque des revendications 1 à 5 **caractérisé en ce que** l'on soumet un composé de formule (II) : 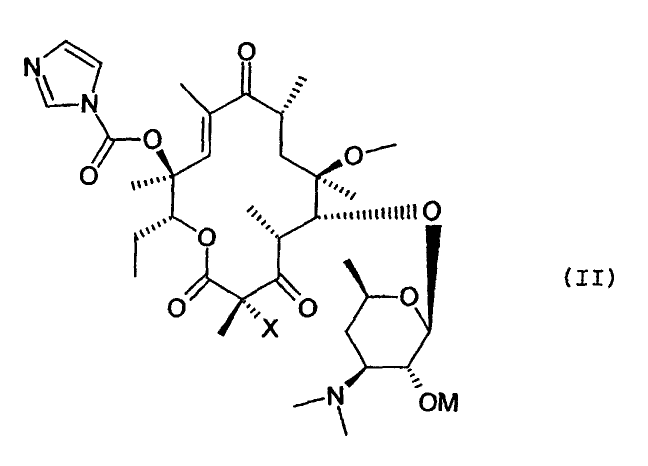 dans laquelle X conserve sa signification précédente et OM représente le reste d'un radical acyle à l'action d'un composé de formule (III) : 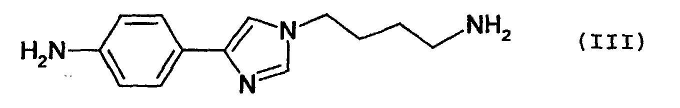 puis si désiré soumet le composé obtenu à l'action d'un agent de déprotection de la fonction hydroxyle en 2' et/ou le cas échéant, à l'action d'un acide pour en former le sel.

7. A titre de produit chimique le composé de formule(III) définie à la revendication 6.

8. A titre de médicament les composés de formule (I) définie à l'une quelconque des revendications 1 à 5 ainsi que leurs sels d'addition avec les acides pharmaceutiquement acceptables.

9. Les compositions pharmaceutiques renfermant comme principe actif au moins un médicament selon la revendication 8.

## Patentansprüche

1. Als chemische Produkte die Verbindungen der Formel (I) sowie ihre Additionssalze mit Säuren: 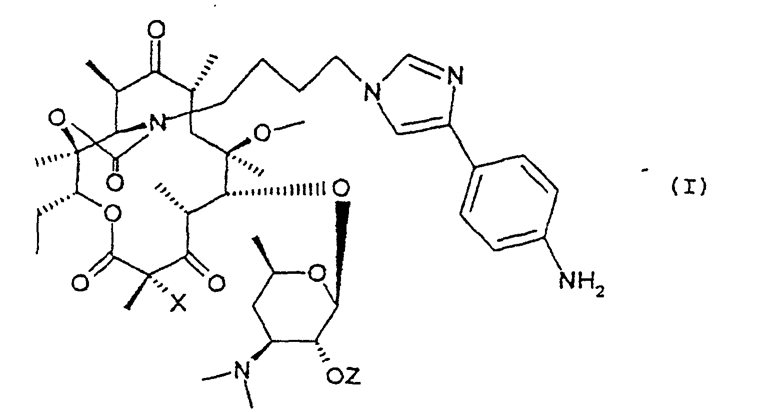 in der X ein Wasserstoffatom oder ein Halogenatom darstellt und Z ein Wasserstoffatom oder der Rest einer Säure bedeutet.

2. Verbindungen der Formel (I), worin X ein Fluoratom ist.

3. Verbindungen der Formel (I) wie in Anspruch 1 oder 2 definiert, worin Z ein Wasserstoffatom ist.

4. Als chemisches Produkt, wie in Anspruch 1 definiert, die folgende Verbindung:
11,12-Dideoxy-3-de-[(2,6-dideoxy-3-C-methyl-3-O-methyl-alpha-Lribohexopyranosyl)-oxy]-6-O-methyl-3-oxo-12,11-{oxycarbonyl-[[4-(4-(4-aminophenyl)-1H-imidazol-1-yl)-butyl]-imino]}-erythromycin.

5. Als chemisches Produkt, wie in Anspruch 1 definiert, die folgende Verbindung:
2-Fluor-11,12-dideoxy-3-de-[(2,6-dideoxy-3-C-methyl-3-O-methylalpha-L-ribohexopyranosyl)-oxy]-6-O-methyl-3-oxo-12,11-{oxycarbonyl-[[4-(4-(4-aminophenyl) -1H-imidazol-1-yl) -butyl] -imino]}-erythromycin.

6. Verfahren zur Herstellung der Verbindungen der Formel (I) wie in irgendeinem der Ansprüche 1 bis 5 definiert, **dadurch gekennzeichnet, daß** man eine Verbindung der Formel (II) 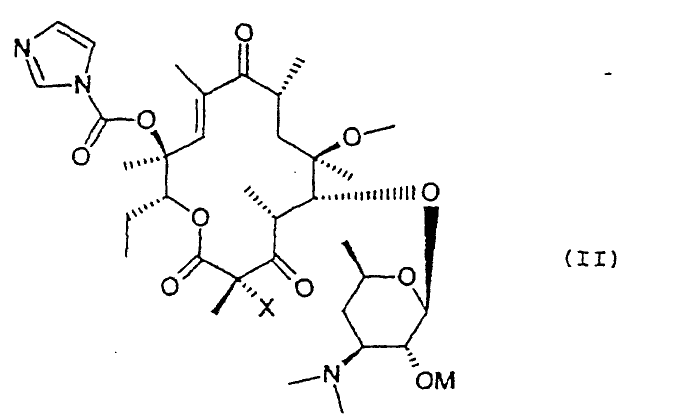 in der X seine vorstehend genannte Bedeutung beibehält und OM den Rest eines Acylrestes darstellt, der Einwirkung einer Verbindung der Formel (III) 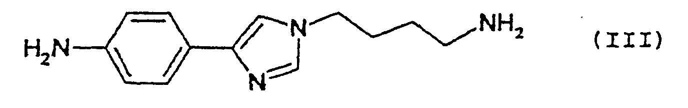 unterzieht, und man anschließend, wenn erwünscht, die erhaltene Verbindung der Einwirkung eines Mittels zum Abspalten der Schutzgruppe von der Hydroxylfunktion in 2' und/oder gegebenenfalls der Einwirkung einer Säure unterwirft, um daraus das Salz zu bilden.

7. Als chemisches Produkt die Verbindung der Formel (III) wie in Anspruch 6 definiert.

8. Als Arzneimittel die Verbindungen der Formel (I) wie in irgendeinem der Ansprüche 1 bis 5 definiert, sowie ihre Additionssalze mit pharmazeutisch akzeptablen Säuren.

9. Pharmazeutische Zusammensetzungen, umfassend als Wirkstoff mindestens ein Arzneimittel wie in Anspruch 8 definiert.

## Claims

1. As chemical products, the compounds of formula (I) 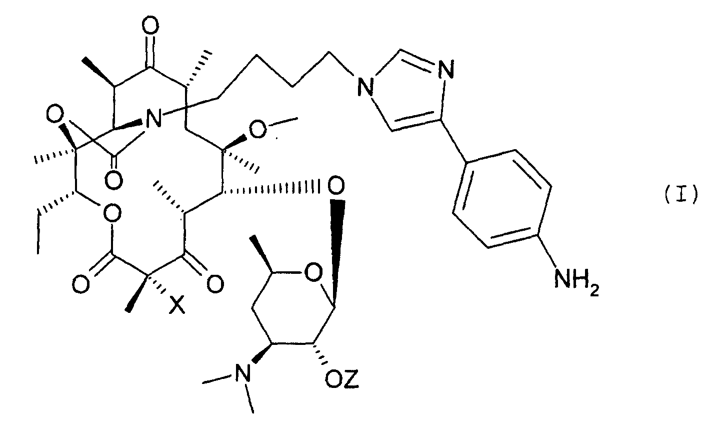 in which X represents a hydrogen atom or a halogen atom and Z represents a hydrogen atom or the remainder of an acid.

2. The compounds of formula (I) in which X represents a fluorine atom.

3. The compounds of formula (I) defined in claim 1 or 2 in which Z represents a hydrogen atom.

4. As a chemical product defined in claim 1, the following compound:
11,12-dideoxy-3-de [(2,6-dideoxy-3-C-methyl-3-O-methyl-alpha-L-ribohexopyranosyl)oxy] 6-0-methyl 3-oxo 12,11-[oxycarbonyl[[4-[4-(4-aminophenyl)-1H-imidazol-1-yl]-butyl]-imino]]-erythromycin.

5. As a chemical product defined in claim 1, the following compound:
2-fluoro-11,12-dideoxy-3-de [(2,6-dideoxy-3-C-methyl-3-O-methyl-alpha-L-ribohexopyranosyl)oxy] 6-0-methyl 3-oxo 12,11-[oxycarbonyl[[4-[4-(4-aminophenyl)-1H-imidazol-1-yl]-butyl]-imino]]-erythromycin.

6. Preparation process for the compounds of formula (I) defined in any one of claims 1 to 5 **characterized in that** a compound of formula (II). 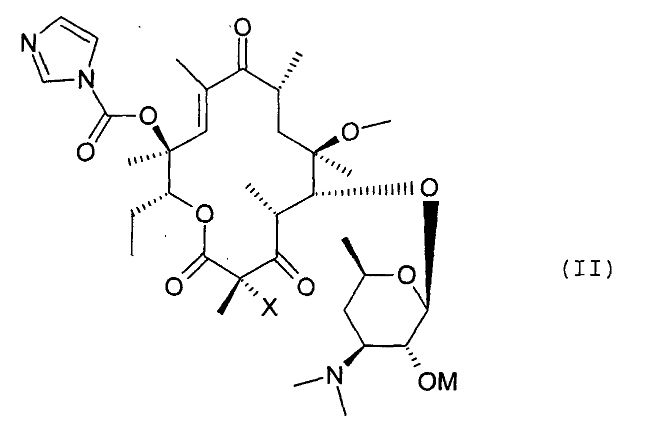 in which X retains its previous meaning and OM represents the remainder of an acyl radical is subjected to the action of a compound of formula (III). 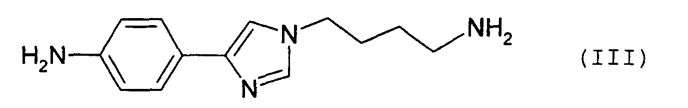 then, if desired, the compound obtained is subjected to the action of a deprotection agent of the hydroxyl function in position 2' and/or if appropriate, to the action of an acid in order form the salt.

7. As a chemical product, the compound of formula (III) defined in claim 6.

8. As a medicament, the compounds of formula (I) defined in any one of claims 1 to 5 as well as their addition salts with pharmaceutically acceptable acids.

9. The pharmaceutical compositions containing at least one medicament according to claim 8 as active ingredient
